# EUROPEAN PATENT SPECIFICATION

(11) **EP 3 965 721 B1**
(45) Date of publication and mention of the grant of the patent: **14.06.2023**
(21) Application number: 20722605.1
(22) Date of filing: 06.05.2020
(51) Int. Cl.: A61K 8/34, A61Q 19/00

(54) **TOPICAL COMPOSITIONS WITH ANTIMICROBIAL AGENT MIXTURE**
TOPISCHE ZUSAMMENSETZUNGEN MIT ANTIMIKROBIELLER WIRKSTOFFMISCHUNG
COMPOSITIONS TOPIQUES COMPRENANT UN MÉLANGE D'AGENTS ANTIMICROBIENS

(30) Priority: 10.05.2019 EP 19173725
(43) Date of publication of application: 16.03.2022
(73) Proprietor: DSM IP Assets B.V., 6411 TE Heerlen (NL)
(72) Inventor: MENDROK-EDINGER, Christine, 4303 Kaiseraugst (CH)
(74) Representative: Berg, Katja
(86) International application number: PCT/EP2020/062567
(87) International publication number: WO 2020/229259

(56) References cited:
- KR-A- 20110 055 829
- US-A1- 2005 131 077
- US-A1- 2016 354 291
- SCHÜLKE: "euxyl PE 9010 Preservative for cosmetics & toiletries", 20121101, 1 November 2012 (2012-11-01), pages 1-6, XP007921278,

## Description

The present invention relates to topical compositions comprising a mixture of phytantriol and a C₄₋₆alkyl glycerine as well as to the use of said mixture for the reduction and/ or prevention of microbial growth.

Microorganisms lead to the decay of food and goods of daily use such as cosmetic compositions, household products, plastics, paper or paints. The reduction of growth of microorganisms is thus essential to enhance shelf life (storage stability) of the product itself but also to assure consumers safety. Thus, generally a preservative is added to the respective products. As the use of certain preservatives is however under strong public discussion, the option of being able to reduce or even avoid such agents is a strong desire in the industries. As cosmetic products are particular prone to be attacked by microorganism, there is accordingly an ongoing need for alternatives to help the cosmetic formula remain safe and prevent alteration in time.

Therefore, the problem to be solved by the present invention is to offer new, safe and efficient ways to reduce or inhibit microbial growth.

Surprisingly it has now been found that the combination of phytantriol and a C₄₋₆alkyl glycerine shows a synergistic antimicrobial effect. This surprising finding is very advantageous as it can be used to improve product stability and shelf life by protecting cosmetic compositions, household products, plastics, paper and/ or paints against fungi (such as yeast and molds) and bacteria. It can also be used to reduce or avoid the amount of preservatives in cosmetic compositions by, nevertheless, maintaining the antimicrobial properties.

Thus, in a first embodiment the present invention relates to topical compositions comprising phytantriol and a C₄₋₆alkyl glycerine.

In a second embodiment, the present invention relates to the use of a mixture of phytantriol and a C₄₋₆alkyl glycerine as a synergistic antimicrobial agent mixture (preservation booster).

In third embodiment, the invention relates to the use of a mixture of phytantriol and a C₄₋₆alkyl glycerine as antimicrobial agent, i.e. an agent which exhibits an antimicrobial activity.

In particular the present invention is directed to the use of a mixture of phytantriol and a C₄₋₆ alkyl glycerine as anti-fungal and/ or anti-bacterial agent, more in particular as an agent for killing and/ or inhibiting the growth of fungi such as in particular yeast or molds and/ or bacteria (gram-positive and/ or gram negative), most preferably of Escherichia Coli (E. Coli), Pseudomonas aeruginosa, (P. aeruginosa), Staphylococcus aureus (S. aureus), Aspergillus brasiliensis (A. brasiliensis), and/ or Candida albicans (C. albicans) as well as mixtures thereof.

In a further embodiment, the invention relates to a method for killing and/ or inhibiting growth of microbial cells, in particular of fungi such as yeast and/ or molds and/ or bacterial cells, most in particular of E. Coli, P. aeruginosa, S. aureus, A. brasiliensis, and/ or C. albicans as well as mixtures thereof, said method comprising contacting said microbial cells with a mixture of phytantriol and a C₄₋₆ alkyl glycerine.

Phytantriol [CAS: 74563-64-7] is a colourless to light yellow, viscous liquid with the chemical name 3,7,11,15-tetramethyl-hexadecane-1,2,3-triol. Phytantriol is e.g. commercially available from DSM Nutritional Products Ltd, Kaiseraugst.

The term C₄₋₆alkyl glycerine refers to linear and cyclic C₄₋₆alkyl glycerines such as butyl glycerine [CAS 624-52-2], pentyl glycerine [CAS 22636-32-4], hexyl glycerine [CAS 10305-38-1], cyclobutyl glycerine, cyclopentyl glycerine and cyclohexyl glycerine [CAS: 10305-41-6], hexyl glycerine (also known as 3-(hexyloxy)-1,2-propanediol) and cyclohexyl glycerine (also known as 3-(cyclohexyloxy)-1,2-propanediol) being particularly preferred. The most preferred C₄₋₆alkyl glycerine in all embodiments of the present invention is hexyl glycerine. Such C₄₋₆alkyl glycerine are e.g. commercially available from Adeka Corporation, Japan.

The term "antimicrobial activity" (or "antimicrobial effect") as used herein means a capability of killing and/or inhibiting the growth of microbial cells such as in particular bacterial and fungal cells (in particular mold and/ or yeast), for example, P. acnes, S. epidermis, M. furfur, A. brasiliensis, E. Coli, P. aeruginosa, S. aureus, A. brasiliensis, and/ or C. albicans as well as mixtures thereof, preferably, however, of E. Coli, P. aeruginosa, S. aureus, A. brasiliensis, and/ or C. albicans as well as mixtures thereof

In all embodiments of the present invention the topical compositions preferably comprise phytantriol in an amount of at least 0.005 wt.-%. More preferably, the amount of phytantriol is selected in the range from about 0.01 wt.-% to 5 wt.-%, more preferably in the range from about 0.05 wt.-% to 3 wt.-% and most preferably in the range from 0.1 wt.-% to 2 wt.-%. Further suitable ranges encompass 0.1 wt.-% to 1.5 wt.-% or 0.1 wt.-% to 1 wt.-%, based on the total weight of the composition.

In all embodiments of the present invention the topical compositions preferably comprise the C₄₋₆alkyl glycerine in an amount (total) selected in the range from about 0.01 wt.-% to about 5 wt.-%, preferably in the range from 0.1 wt.-% to 4 wt.-%, most preferably in the range of 0.25 wt.-% to 3 wt.-%, based on the total weight of the composition. Further suitable ranges encompass 0.25 wt.-% to 2 wt.-% and 0.3 wt.-% to 1 wt.-%.

The weight-ratio (w/w) of the C₄₋₆alkyl glycerine to phytantriol in all embodiments of the present invention is preferably selected in the range from 5:1 to 1:5, such as in the range from 2.5:1 to 1:2.5, such as most preferable in the range from 1.5:1 to 1:1. Most preferred, in all embodiments of the present invention the C₄₋₆alkyl glycerine is used in excess, i.e. an amount higher than the amount of phytantriol such as in a weight-ratio larger than 1, more preferably in a weight-ratio of at least 1.25 (i.e. 1.25 parts of C₄₋₆alkyl glycerine to1 part of phytantriol).

To make use of the synergistic anti-microbial activity of the mixture of phytantriol and C₄₋₆alkyl glycerine, it can be used in a multiplicity of formulations or applications, such as, for example, cosmetic or pharmaceutical compositions, medicinal products or household products.

On the one hand, the use according to the present invention can be a technical use such as to reduce or prevent microbial growth in topical compositions, for example to enhance shelf-life and/ or storage stability.

On the other hand, the use according to the invention of the mixture of phytantriol and a C₄₋₆alkyl glycerine can take place in a cosmetic or pharmaceutical sense. A pharmaceutical application is conceivable, for example, in the case of a specific anti-microbial therapy. A cosmetic application is conceivable, for example, for maintaining a healthy skin homeostasis and/ or for balancing the skin microbiome.

The mixture of phytantriol and a C₄₋₆alkyl glycerine (or a topical composition comprising said mixture according to the present invention) can also be used for the treatment and/ or prophylaxis of pathological skin conditions such as dermatitis and/ or acne by maintaining a healthy skin homeostasis and/ or by balancing the skin microbiome, e.g. by avoiding the (excessive) growth of pathological microbes, without being bound thereto by theory.

In all embodiments of the present invention, the methods and uses according to the present invention are preferably technical and/ or cosmetic (non-therapeutic) most preferably technical (i.e. for preservation purposes).

However, the present invention also relates in another aspect to the topical composition according to the invention for the use in the treatment and/ or prophylaxis of pathological skin conditions such as dermatitis and/ or acne by maintaining a healthy skin homeostasis and/ or by balancing the skin microbiome, said method preferably comprising the steps of contacting the skin and/ or scalp with said topical composition.

The topical compositions according to the present invention are preferably cosmetic or pharmaceutical compositions which are topically applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The cosmetic or pharmaceutical compositions according to the present invention preferably further comprise a physiologically acceptable medium, that is to say a medium compatible with keratinous substances, such as the skin, mucosa, and keratinous fibers. Preferably, the physiologically acceptable medium is a cosmetically or pharmaceutically acceptable carrier.

The term cosmetically or pharmaceutically acceptable carrier refers to all carriers and/or excipients and/ or diluents conventionally used in cosmetic compositions such as in particular oils (cosmetic oils), water, surfactants, emulsifiers, thickeners.

The topical compositions according to the present invention are generally prepared by admixing phytantriol and the C₄₋₆alkyl glycerine in the amounts indicated herein with a suitable carrier.

The exact amount of carrier will depend upon the actual level of phytantriol and the C₄₋₆alkyl glycerine and any other optional ingredients that one of ordinary skill in the art would classify as distinct from the carrier (e.g., other active ingredients).

In an advantageous embodiment, the cosmetic or pharmaceutical compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic or pharmaceutical compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Preferably, the cosmetic or pharmaceutical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

The cosmetic or pharmaceutical compositions in accordance with the invention can be in the form of a liquid, lotion, a thickened lotion, a gel, a cream, a milk, an ointment or a paste.

The cosmetic or pharmaceutical compositions according to the invention have a pH in the range of 3-10, preferably in the range of pH of 3-8, most preferred in the range of pH 3-7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention the acid, if present, is used in an amount of at least 0.0001 wt.-%, such as e.g. in an amount of 0.01-1 wt.-%, in particular in an amount of 0.01 to 0.5 wt.-%.

The cosmetic compositions according to the present invention advantageously comprise an additional preservative or preservation booster. Particular suitable preservatives or preservation booster in all embodiments of the present invention are benzoic acid, sodium benzoate, sorbic acid, potasssium sorbate, dehydroacetic acid, caprylhydroxamic acid, alcohol, alcohol denat., hydroxyacetophenone, caprylyl glycol, pentylene glycol, 1,2-hexanediol, decylene glycol, monoglycerides such as glyceryl laurate, propylene glycol caprylate, propylene glycol heptanoate as well as mixtures thereof. When present, the preservative and/ or preservation booster is preferably used in an amount of 0.01 to 2 wt.-%, more preferably in an amount of 0.05 to 1.5 wt.-%, most preferably in an amount of 0.1 to 1.0 wt.-%, based on the total weight of the composition.

It is furthermore preferred that the topical compositions according to the present invention are free of any parabenes, benzethoniumchlorid, piroctone olamine, lauroylarginat, methylisothiazolinon, chlormethylisothiazolinon, bronopol, benzalkoniumchloride, formaldeh releasing compounds, salicylic acid, triclosan, DMDM hydantoin, chlorphenesin and IPBC (lodopropinylbutyl carbamate).

The cosmetic compositions according to the present invention are in particular skin care preparations, functional preparations and/ or hair care preparations such as most in particularly skin or hair care preparations.

Examples of skin care preparations are, in particular, light protective preparations (sunscreen preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples of hair care preparations which are suitable according to the invention and which may be mentioned are shampoos, hair conditioners (also referred to as hair rinses, hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

If the hair care preparations according to the invention are supplied as shampoos, these can be clear liquids, opaque liquids (with pearly luster effect), in cream form, gel-like or else in powder form or in tablet form, and as aerosols. The surfactant raw materials on which these shampoos are based can be anionic, cationic, nonionic and amphoteric in nature and also be present in combinations of these substances.

Examples of anionic surfactants suitable for the incorporation into the shampoo preparations according to the present invention are C₁₀₋₂₀ alkyl- and alkylenecarboxylates, alkyl ether carboxylates, fatty alcohol sulfates, fatty alcohol ether sulfates, alkylolamide sulfates and sulfonates, fatty acid alkylolamide polyglycol ether sulfates, alkanesulfonates and hydroxyalkanesulfonates, olefinsulfonates, acyl esters of isothionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyltaurides, fatty acid sarcosinates, and sulforicinoleates. These compounds and their mixtures are used in the form of their salts which are soluble in water or dispersible in water, for example the sodium, potassium, magnesium, ammonium, mono-, di- and triethanolammonium and analogous alkylanunonium salts.

Examples of suitable cationic surfactants are quaternary ammonium salts such as di(C₁₀₋-C₂₄alkyl)dimethylammonium chloride or bromide, preferably di (C₁₂-C₁₈alkyl)-dimethylammonium chloride or bromide; C₁₀-C₂₄-alkyldimethylethylammonium chloride or bromide; C₁₀-C₂₄-alkyltrimethylammonium chloride or bromide, preferably cetyltrimethylammonium chloride or bromide and C₂₀-C₂₄-alkyltrimethylammonium chloride or bromide; C₁₀-C₂₄4 -alkyldimethylbenzylammonium chloride or bromide, preferably C₁₂-C₁₈-alkyldime methylbenzylammoniumchloride; N-(C₁₂-C₁₈-alkyl)pyridinium chloride or bromide, preferably N- (C₁₂-C₁₆-alkyl)pyridinium chloride or bromide; N-(C₁₂-C₁₈-alkyl)isoquinolinium chloride, bromide or monoalkyl sulfate; N-(C₁₂-C₁₈-alkyloylcolaminoformylmethyl)pyridinium chloride; N-(C₁₂-C₁₈-alkyl)-N-methylmorpholinium chloride, bromide or monoalkyl sulfate; N-(C₁₂-C₁₈-alkyl)-N-ethylmorpholinium chloride, bromide or monoalkyl sulfate; C₁₆-C₁₈-alkylpentaoxethylammonium chloride; isobutylphenoxyethoxyethyldimethyl-benzylammonium chloride; salts of N,N-diethylaminoethylstearylamide and oleylamide with hydrochloric acid, acetic acid, lactic acid, citric acid, phosphoric acid; N-acylamidoethyl-N,N-diethyl-N-methylammonium chloride, bromide or monoalkylsulfate and N-acylaminoethyl-N,N-diethyl-N-benzylammonium chloride, bromide or monoalkyl sulfate, where acyl is preferably stearyl or oleyl.

Examples of suitable nonionic surfactants which can be used as detergent substances are fatty alcohol ethoxylates (alkylpolyethylene glycols); alkylphenol polyethylene glycols; alkyl mercaptan polyethylene glycols; fattyamine ethoxylates (alkylaminopolyethylene glycols); fatty acid ethoxylates (acylpolyethylene glycols); polypropylene glycol ethoxylates (Pluronic); fatty acid alkylolamides (fatty acid amide polyethylene glycols); sucrose esters; sorbitol esters and polyglycol ether.

Examples of amphoteric surfactants which can be added to the shampoos are N-(C₁₂-C₁₈-alkyl)-.beta.-aminopropionates and N-(C₁₂-C₁₈-alkyl)-.beta.-iminodipropionates as alkali metal and mono-, di- and trialkylammonium salts; N-acylamidoalkyl-N,N-dimethylacetobetaine, preferably N-(C₈-C₁₈-acyl)amidopropyl-N, N-dimethylacetobetaine; C₁₂-C₁₈-alkyldimethylsulfopropylbetaine; amphoteric surfactants based on imidazoline (commercial name: Miranol^{®}, Steinapon^{®}), preferably the sodium salt of 1-(β-carboxymethyloxyethyl)-1-(carboxymethyl)-2-laurylimidazolinium; amine oxide, for example C₁₂-C₁₈-alkyldimethylamine oxide, fatty acid amidoalkyldimethylamine oxide.

The hair care preparations according to the invention can additionally contain further additives customary in hair care such as for example perfumes, colorants, also those which simultaneously dye or tint the hair, solvents, opacifying agents and pearly luster agents, for example esters of fatty acids with polyols, magnesium and zinc salts of fatty acids, dispersions based on copolymers, thickening agents such as sodium, potassium and ammonium chloride, sodium sulfate, fatty acid alkylolamides, cellulose derivatives, natural rubbers, also plant extracts, protein derivatives such as gelatin, collagen hydrolysates, polypeptides with a natural or synthetic basis, egg yolk, lecithin, lanolin and lanolin derivatives, fats, oils, fatty alcohols, silicones, deodorizing agents, substances with antimicrobial activity, substances with antiseborrhoeic activity, substances with keratolytic and keratoplastic effect, such as, for example, sulfur, salicylic acid and enzymes as well as further anti-dandruff agents such as olamine, climbazol, zink pyrithion, ketoconazole, salicylic acid, sulfur, tar preparations, derivatives of undecenic acid, extracts of nettel, rosmary, cottonwood, birch, walnut, willow bark and/ or arnica.

The topical cosmetic compositions according to the present invention are advantageously O/W emulsions, W/O emulsions and/ or gels such as shower gels.

Furthermore, the topical compositions in the form of O/W emulsions, W/O emulsions and/ or gels according to the present invention are skin care preparation intended for the treatment of acne or for maintaining a healthy skin homeostasis and/ or for maintaining skin microbiome balance.

O/W emulsions according to the present invention advantageously comprise (i) phytantriol in an amount selected in the range of about 0.01 to 2 wt.-%, preferably in the range of 0.05 to 1.5 wt.-%, most preferably in the range of 0.1 to 1 wt.-%, based on the total weight of the composition, (ii) a C₄₋₆alkyl glycerine in an amount selected in the range of about 0.01 to about 2 wt.-%, preferably in the range of 0.1 to 1.5 wt.-%, most preferably in the range of 0.1 to 1 wt.-%, based on the total weight of the composition, (iii) water and (iv) at least one O/W- or Si/W-emulsifier selected from the list of glycerylstearatcitrate, glycerylstearate (self-emulsifying), stearic acid, salts of stearic acid, polyglyceryl-3-methylglycosedistearate, ceteareth-20, steareth-2, steareth-12, PEG-40 stearate, phosphate esters and the salts thereof such as cetyl phosphate (Amphisol^{®} A), diethanolamine cetyl phosphate (Amphisol^{®} DEA), potassium cetyl phosphate (Amphisol^{®} K), sodiumcetearylsulfat, sodium glyceryl oleate phosphate, hydrogenated vegetable glycerides phosphate, sorbitan oleate, sorbitan sesquioleate, sorbitan isostearate, sorbitan trioleate, lauryl glucoside, decyl glucoside, sodium stearoyl glutamate, sucrose polystearate and Hydrated Polyisobuten as well as mixtures thereof. Also, one or more synthetic polymers may be used as an emulsifier such as for example, PVP eicosene copolymer, acrylates/C10-3o alkyl acrylate crosspolymer, acrylates/steareth-20 methacrylate copolymer, PEG-22/dodecyl glycol copolymer, PEG-45/dodecyl glycol copolymer, and mixtures thereof. In a particular preferred embodiment the O/W-emulsifier is selected from the group of cetyl phosphates such as in particular potassium cetyl phosphate (commercially available as Amphisol^{®} K), glyceryl stearate (and) PEG 100 stearate (commercially available as Arlacel^{®} 165) and/ or polyalkylenglycolether such as in particular laureth-35 (lauryl alcohol with 35 EO units; commercially available as Brij^{®} 35).The at least one O/W emulsifier is preferably used in an amount of about 0.001 to 10 wt.-%, more preferably in an amount of 0.1 to 7 wt.-% with respect to the total weigh of the composition. Additionally, the cosmetic composition in the form of a O/W emulsion contains advantageously at least one co-emulsifier selected from the list of alkyl alcohols such as Cetyl Alcohol (Lorol C16, Lanette 16) Cetearyl Alcohol (Lanette^{®} O), Stearyl Alcohol (Lanette^{®} 18), Behenyl Alcohol (Lanette^{®} 22), Glyceryl Monostearate, Glyceryl Myristate (Estol^{®} 3650), Hydrogenated Coco- Glycerides (Lipocire Na10) without being limited to this and mixtures thereof.

W/O emulsions according to the present invention advantageously comprise (i) phytantriol in an amount selected in the range 0.01 to 2 wt.-%, preferably in the range of 0.05 to 1.5 wt.-%, most preferably in the range of 0.1 to 1 wt.-%, based on the total weight of the composition, (ii) a C₄₋₆alkyl glycerine in an amount selected in the range of about 0.01 to about 2 wt.-%, preferably in the range of 0.1 to 1.5 wt.-%, most preferably in the range of 0.1 to 1 wt.-%, based on the total weight of the composition, (iii) water and (iv) at least one W/O- or W/Si-emulsifier selected from the list of polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

Gel preparations according to the present invention advantageously comprise (i) phytantriol in an amount selected in the range of 0.01 to 2 wt.-%, preferably in the range of 0.05 to 1.5 wt.-%, most preferably in the range of 0.1 to 1 wt.-%, based on the total weight of the composition, (ii) a C₄₋₆alkyl glycerine in an amount selected in the range of about 0.01 to about 2 wt.-%, preferably in the range of 0.1 to 1.5 wt.-%, most preferably in the range of 0.1 to 1 wt.-%, based on the total weight of the composition, (iii) water and (iv) at least one water soluble thickener. Such water-soluble thickeners are well known to a person skilled in the art and are e.g. listed in the "Handbook of Water soluble gums and resins" by Robert L. Davidson (Mc Graw Hill Book Company (1980)). Particularly suitable water soluble thickeners are selected from the group consisting of polyacrylic acids (e.g. commercially available under the tradename Carbomer or Carbopol^{®}), homopolymers of 2-Acrylamido-2-methylpropansulfonic acid (e.g. commercially available as Rheothik^{®}11-80), acrylate copolymers (e.g. commercially available under the tradename Pemulen^{®} or Aculyn^{®} 33), branched Poly(methacryloyloxyethyltrimethylammoniumchlorid) (INCI-name Polyquaternium-37), non-modified guar gums (e.g. commercially available under the tradename Jaguar), starch or derivatives thereof and/ or hydroxyalkylcellulosen. Preferably the water-soluble thickener is used in an amount of about 0.001 to 10 wt.-%, more preferably in an amount of 0.2 to 7 wt.-%, based on the total weigh of the composition.

It has been observed that, when adding the phytanrtriol with a C₄₋₆alkyl glycerine to a topical composition, the antimicrobial action is increased significantly. In other words, by adding the synergistic antimicrobial agent mixture to a composition, a smaller number of microbes are observed to grow than without the use of phytantriol or the use of C₄₋₆alkyl glycerine., i.e. with phytantriol or a C₄₋₆alkyl glycerine alone. Phytantriol has, hence, a synergistic effect on the antimicrobial action of a C₄₋₆alkyl glycerine, particularly in a cosmetic composition.

This is very advantageous also in as accordingly the amount of the C₄₋₆alkyl glycerine can be reduced by adding an effective amount of phytantriol while maintaining the same antimicrobial action.

It has been further observed that the antimicrobial action is particularly pronounced in aqueous systems. This is very advantageous as it is known that it is typically the water phase of a product which is most susceptible to microbial growth.

In a further aspect, the invention relates to a method of inhibiting or delaying microbial breakdown of a cosmetic composition, household product, plastic, paper and/ or paint, wherein said method comprises the step of adding to the cosmetic composition, household product, plastic, paper and/ or paint a mixture of phytantriol and C₄₋₆alkyl glycerine in an amount of
(i) 0.01 to 2 wt.-%, preferably of 0.05 to 1.5 wt.-%, most preferably of 0.1 to 1 wt.-% of phytantriol, based on the total weight of the composition, and
(ii) 0.01 to 2 wt.-%, preferably of 0.1 to 1.5 wt.-%, of 0.1 to 1 wt.-%, of a C₄₋₆alkyl glycerine, based on the total weight of the composition.

The following examples are provided to further illustrate the compositions and effects of the present invention. These examples are illustrative only and are not intended to limit the scope of the invention in any way.

### Example

The evaluation of the preservation of a cosmetic formulation is based on inoculation of the formulation as outlined in table 1 with calibrated inocula (prepared from relevant strains of micro-organisms). Therefore, to each formulation 0.2 ml of calibrated inoculum is added to obtain between 1×10⁴ cfu/ml and 1×10⁷ cfu/ml or g for bacteria, and between 1×10² cfu/ml and 1×10⁶ cfu/ml or g for C. albicans and A. brasiliensis in the formulation (final concentration), which is mixed thoroughly into the formulation to ensure a homogeneous distribution of the inoculum. Then the respective containers containing the inoculated formulations are stored @ 22.5 ± 2.5°C. At each specified sampling interval (initial (T0), 7 days (T7) and 28 days (T28)) 1g of the inoculated formulation is taken and a dilution prepared to perform the enumeration of surviving micro-organisms. Then, for each time and each strain/ strain mixture, the average log value is calculated which is represented in tables 2-6. (Procedure: see ISO 11930, chapter 5.2. - 5.6.3.4. (page 3 - 13))

**Table 1: O/W emulsion**

| | **Ingredients (INCI)** | | ***Wt.-%*** |
|---|---|---|---|
| A | Potassium Cetyl Phosphate | | 2.0 |
| | Cetearyl Alcohol | | 3.0 |
| | Cetyl Alcohol | | 2.0 |
| | Dimethicone | | 3.0 |
| | C12-15 Alkyl Benzoate | | 15.0 |
| B | Xanthan Gum | | 0.3 |
| | Glycerin | | 3.0 |
| | Aqua | | Ad 100 |
| | Active(s): | Phytantriol (PTT) and/or Hexylglycerin (HG) and/or Ethylhexyl glycerine (EHG) | Amounts as indicated in the results outlined below |

**Table 2: Results for C. albicans; PTT: 0.4 wt.-%, HG & EHG: 0.6 wt.-%**

| # | *PTT (Reference)* | *HG (Reference)* | *EHG (Reference)* | *PTT* & *HG (Invention)* | *PTT* & *EHG (Reference)* |
|---|---|---|---|---|---|
| *Time [days]* | Average log [cfu/ml] | | | | |
| 0 | 3.0 | 4.3 | 3.2 | 2.5 | 3.5 |
| 7 | 6.8 | 3.6 | 6.1 | 0 | 6.2 |
| 28 | 7.1 | 3.1 | 6.0 | 0 | 6.3 |

As can be seen in the table above the combination of phytantriol and hexyl glycerine exhibits a synergistic effect against Candida albicans. The combination also performs better than a combination of phytantriol and ethylhexyl glycerine (a well-known preservation booster).

**Table 3: Results for A. brasiliensis; PTT: 0.1 wt.-%, HG & EHG: 0.9 wt.-%**

| # | *PTT (Reference)* | *HG (Reference)* | *EHG (Reference)* | *PTT* & *HG (Invention)* | *PTT* & *EHG (Reference)* |
|---|---|---|---|---|---|
| *Time [days]* | Average log [cfu/ml] | | | | |
| *0* | 2.1 | 4.2 | 4.6 | 4.5 | 4.4 |
| *7* | 3.6 | 1.3 | 4.2 | 0 | 4.5 |
| *28* | 4.4 | 0 | 4.5 | 0 | 3.6 |

**Table 4: Results for A. brasiliensis; PTT: 0.4 wt.-%, HG & EHG: 0.6 wt.-%**

| # | *PTT (Reference)* | *HG (Reference)* | *EHG (Reference)* | *PTT* & *HG (Invention)* | *PTT* & *EHG (Reference)* |
|---|---|---|---|---|---|
| *Time [days]* | Average log [cfu/ml] | | | | |
| *0* | 1.7 | 4.3 | 4.6 | 4.7 | 4.7 |
| *7* | 3.5 | 3.6 | 4.4 | 1.9 | 4.5 |
| *28* | 4.5 | 3.1 | 4.6 | 0 | 4.4 |

**Table 5: A. brasiliensis; PTT: 0.3 wt.-%, HG & EHG: 0.4 wt.-%**

| # | *PTT (Reference)* | *HG (Reference)* | *EHG (Reference)* | *PTT* & *HG (Invention)* | *PTT* & *EHG (Reference)* |
|---|---|---|---|---|---|
| *Time [days]* | Average log [cfu/ml] | | | | |
| *0* | 6.3 | 4.7 | 4.6 | 3.9 | 4.8 |
| *7* | 5.7 | 3.7 | 4.6 | 2.1 | 4.2 |
| *28* | 5.8 | 3.5 | 4.6 | 2.0 | 4.3 |

As can be retrieved from table 3 to 5, the combination of phytantriol and hexyl glycerine at different concentration levels/ weight ratios shows a synergistic effect against Aspergillus brasiliensis. The combination performs better than the combination of phytantriol and ethylhexyl glycerine (a well-known preservation booster).

**Table 6: Bacterial cocktail*; PTT: 0.3 wt.-%, HG & EHG: 0.4 wt.-%**

| # | *PTT (Reference)* | *HG (Reference)* | *EHG (Reference)* | *PTT* & *HG (Invention)* | *PTT* & *EHG (Reference)* |
|---|---|---|---|---|---|
| *Time [days]* | Average log [cfu/ml] | | | | |
| *0* | 6.5 | 4.9 | 5.3 | 4.6 | 4.3 |
| *7* | 2 | 5.7 | 7.0 | 0 | 7.3 |
| *28* | 2 | 5.6 | 6.5 | 0 | 7.6 |

| | | | | | |
|---|---|---|---|---|---|
| **E. Coli, P. aeruginosa, S*. *aureus* | | | | | |

As can be retrieved from table 6, the combination of phytantriol and hexyl glycerine also exhibits a synergistic effect against bacteria. The combination performs better than the combination of phytantriol andethylhexyl glycerine (a well-known preservation booster).

## Claims

1. A topical composition comprising phytantriol and a C₄₋₆alkyl glycerine.

2. The topical composition according to claim 1, wherein the amount of phytantriol is selected in the range from 0.01 wt.-% to 5 wt.-%, preferably in the range from 0.05 wt.-% to 3 wt.-%, most preferably in the range from 0.1 wt.-% to 2 wt.-%, based on the total weight of the composition.

3. The topical composition according to claim 1 or 2, wherein the amount of the C₄₋₆alkyl glycerine is selected in the range from 0.01 wt.-% to 5 wt.-%, preferably in the range from 0.1 wt.-% to 4 wt.-%, most preferably in the range from 0.25 wt.-% to 3 wt.-%, based on the total weight of the composition.

4. The topical composition according to anyone of the preceding claims, wherein the weight-ratio of the C₄₋₆alkyl glycerine to phytantriol is selected in the range from 5:1 to 1:5, preferably from 2.5:1 to 1:2.5, most preferable from 1.5:1 to 1:1.

5. The topical composition according to anyone of the preceding claims, wherein the C₄₋₆alkyl glycerine is hexyl glycerine or cyclohexyl glycerine, preferably hexyl glycerine.

6. The topical composition according to any one of the preceding claims, wherein the composition is a cosmetic or pharmaceutical composition.

7. The topical composition according to claim 6, wherein the composition is a shampoo preparation, a hair conditioner, an O/W emulsion, a W/O emulsion or a gel.

8. The topical composition according to any one of the preceding claims, wherein the composition comprises water and at least one agent selected from the group consisting of surfactants, emulsifiers, thickeners and oils.

9. A non-therapeutic method of treating the skin and/ or the scalp, said method comprising the steps of contacting the skin and/ or scalp with a topical composition according to anyone of claim 1 to 8.

10. The topical composition according to any one of claim 1 to 8 for the prevention and/ or treatment of pathological skin conditions by maintaining a healthy skin homeostasis and/ or for maintaining skin microbiome balance.

11. Use of a mixture of phytantriol and a C₄₋₆alkyl glycerine as a synergistic antimicrobial agent mixture.

12. The use according to claim 11, wherein the C₄₋₆alkyl glycerine is hexyl glycerine or cyclohexyl glycerin, preferably hexyl glycerine.

13. Use of a mixture of phytantriol and a C₄₋₆alkyl glycerine to reduce or prevent microbial growth in a topical composition according to anyone of claims 1 to 8 upon storage.

14. The use according to claim 13, to enhance shelf life and/ or storage stability of the composition.

15. A method of inhibiting or delaying microbial breakdown of a cosmetic composition, household product, plastic, paper and/ or paint, wherein said method comprises the step of adding to the cosmetic composition, household product, plastic, paper and/ or paint a mixture of phytantriol and C₄₋₆alkyl glycerine in an amount of
(i) 0.01 to 2 wt.-%, preferably of 0.05 to 1.5 wt.-%, most preferably of 0.1 to 1 wt.-%, based on the total weight of the composition, of phytantriol, and
(ii) 0.01 to 2 wt.-%, preferably of 0.1 to 1.5 wt.-%, of 0.1 to 1 wt.-%, based on the total weight of the composition, of a C₄₋₆alkyl glycerine.

## Patentansprüche

1. Topische Zusammensetzung, umfassend Phytantriol und ein C₄₋₆-Alkylglycerin.

2. Topische Zusammensetzung nach Anspruch 1, wobei die Menge an Phytantriol im Bereich von 0,01 Gew.-% bis 5 Gew.-%, vorzugsweise im Bereich von 0,05 Gew.-% bis 3 Gew.-%, ganz besonders bevorzugt im Bereich von 0,1 Gew.-% bis 2 Gew.-%, bezogen auf das Gesamtgewicht der Zusammensetzung, ausgewählt ist.

3. Topische Zusammensetzung nach Anspruch 1 oder 2, wobei die Menge des C₄₋₆-Alkylglycerins im Bereich von 0,01 Gew.-% bis 5 Gew.-%, vorzugsweise im Bereich von 0,1 Gew.-% bis 4 Gew.-%, ganz besonders bevorzugt im Bereich von 0,25 Gew.-% bis 3 Gew.-%, bezogen auf das Gesamtgewicht der Zusammensetzung, ausgewählt ist.

4. Topische Zusammensetzung nach einem der vorhergehenden Ansprüche, wobei das Gewichtsverhältnis von C₄₋₆-Alkylglycerin zu Phytantriol im Bereich von 5:1 bis 1:5, vorzugsweise von 2,5:1 bis 1:2,5, weiter bevorzugt von 1,5:1 bis 1:1, ausgewählt ist.

5. Topische Zusammensetzung nach einem der vorhergehenden Ansprüche, wobei es sich bei dem C₄₋₆-Alkyl-glycerin um Hexylglycerin oder Cyclohexylglycerin, vorzugsweise Hexylglycerin, handelt.

6. Topische Zusammensetzung nach einem der vorhergehenden Ansprüche, wobei es sich bei der Zusammensetzung um eine kosmetische oder pharmazeutische Zusammensetzung handelt.

7. Topische Zusammensetzung nach Anspruch 6, wobei es sich bei der Zusammensetzung um eine Shampoozubereitung, ein Haarkonditionierungsmittel, eine O/W-Emulsion, eine W/O-Emulsion oder ein Gel handelt.

8. Topische Zusammensetzung nach einem der vorhergehenden Ansprüche, wobei die Zusammensetzung Wasser und mindestens ein Mittel aus der Gruppe bestehend aus Tensiden, Emulgatoren, Verdickern und Ölen umfasst.

9. Nichttherapeutisches Verfahren zur Behandlung der Haut und/oder der Kopfhaut, wobei das Verfahren die Schritte des Inkontaktbringens der Haut und/oder Kopfhaut mit einer topischen Zusammensetzung nach einem der Ansprüche 1 bis 8 umfasst.

10. Topische Zusammensetzung nach einem der Ansprüche 1 bis 8 zur Prävention und/oder Behandlung von pathologischen Hautzuständen durch Aufrechterhaltung einer gesunden Hauthomöostase und/oder zur Aufrechterhaltung der Hautmikrobiombalance.

11. Verwendung einer Mischung von Phytantriol und einem C₄₋₆-Alkylglycerin als synergistische Mischung von antimikrobiellen Mitteln.

12. Verwendung nach Anspruch 11, wobei es sich bei dem C₄₋₆-Alkylglycerin um Hexylglycerin oder Cyclohexylglycerin, vorzugsweise Hexylglycerin, handelt.

13. Verwendung einer Mischung von Phytantriol und einem C₄₋₆-Alkylglycerin zur Verringerung oder Prävention von mikrobiellem Wachstum in einer topischen Zusammensetzung nach einem der Ansprüche 1 bis 8 bei der Lagerung.

14. Verwendung nach Anspruch 13 zur Verbesserung der Haltbarkeit und/oder Lagerstabilität der Zusammensetzung.

15. Verfahren zur Inhibierung oder Verzögerung des mikrobiellen Abbaus einer kosmetischen Zusammensetzung, eines Haushaltsprodukts, eines Kunststoffs, eines Papiers und/oder eines Anstrichmittels, wobei das Verfahren den Schritt des Zugebens einer Mischung von Phytantriol und einem C₄₋₆-Alkylglycerin in einer Menge von
(i) 0,01 bis 2 Gew.-%, vorzugsweise 0,05 bis 1,5 Gew.-%, ganz besonders bevorzugt 0,1 bis 1 Gew.-%, bezogen auf das Gesamtgewicht der Zusammensetzung, Phytantriol und
(ii) 0,01 bis 2 Gew.-%, vorzugsweise 0,1 bis 1,5 Gew.-%, 0,1 bis 1 Gew.-%, bezogen auf das Gesamtgewicht der Zusammensetzung, eines C₄₋₆-Alkylglycerins
zu der kosmetischen Zusammensetzung, dem Haushaltsprodukt, dem Kunststoff, dem Papier und/oder dem Anstrichmittel umfasst.

## Revendications

1. Composition topique comprenant du phytantriol et une C₄₋₆alkyl-glycérine.

2. Composition topique selon la revendication 1, la quantité de phytantriol étant choisie dans la plage de 0,01 % en poids à 5 % en poids, préférablement dans la plage de 0,05 % en poids à 3 % en poids, le plus préférablement dans la plage de 0,1 % en poids à 2 % en poids, sur la base du poids total de la composition.

3. Composition topique selon la revendication 1 ou 2, la quantité de la C₄₋₆alkyl-glycérine étant choisie dans la plage de 0,01 % en poids à 5 % en poids, préférablement dans la plage de 0,1 % en poids à 4 % en poids, le plus préférablement dans la plage de 0,25 % en poids à 3 % en poids, sur la base du poids total de la composition.

4. Composition topique selon une quelconque des revendications précédentes, le rapport en poids de la C₄₋₆alkyl-glycérine sur le phytantriol étant choisi dans la plage de 5 : 1 à 1 : 5, préférablement de 2,5 : 1 à 1 : 2,5, le plus préférablement de 1,5 : 1 à 1 : 1.

5. Composition topique selon une quelconque des revendications précédentes, la C₄₋₆alkyl-glycérine étant l'hexyl-glycérine ou la cyclohexyl-glycérine, préférablement l'hexyl-glycérine.

6. Composition topique selon une quelconque des revendications précédentes, la composition étant une composition cosmétique ou pharmaceutique.

7. Composition topique selon la revendication 6, la composition étant une préparation de shampooing, un après-shampooing capillaire, une émulsion H/E, une émulsion E/H ou un gel.

8. Composition topique selon une quelconque des revendications précédentes, la composition comprenant de l'eau et au moins un agent choisi dans le groupe constitué par des tensioactifs, des émulsifiants, des épaississants et des huiles.

9. Procédé non thérapeutique de traitement de la peau et/ou du cuir chevelu, ledit procédé comprenant les étapes de mise en contact de la peau et/ou du cuir chevelu avec une composition topique selon l'une quelconque des revendications 1 à 8.

10. Composition topique selon l'une quelconque des revendications 1 à 8 pour la prévention et/ou le traitement d'affections cutanées pathologiques en maintenant une homéostasie cutanée saine et/ou pour le maintien de l'équilibre du microbiome cutané.

11. Utilisation d'un mélange de phytantriol et d'une C₄₋₆alkyl-glycérine en tant que mélange d'agents antimicrobiens synergistiques.

12. Utilisation selon la revendication 11, la C₄₋₆alkyl-glycérine étant l'hexyl-glycérine ou la cyclohexyl-glycérine, préférablement l'hexyl-glycérine.

13. Utilisation d'un mélange de phytantriol et d'une C₄₋₆alkyl-glycérine pour réduire ou prévenir une croissance microbienne dans une composition topique selon l'une quelconque des revendications 1 à 8 au stockage.

14. Utilisation selon la revendication 13, pour accroître la durée de vie et/ou la stabilité au stockage de la composition.

15. Procédé d'inhibition ou de retardement d'une dégradation microbienne d'une composition cosmétique, d'un produit ménager, d'une matière plastique, de papier et/ou d'une peinture, ledit procédé comprenant l'étape d'ajout à la composition cosmétique, au produit ménager, à la matière plastique, au papier et/ou à la peinture d'un mélange de phytantriol et d'une C₄₋₆alkyl-glycérine en une quantité de
(i) 0,01 à 2 % en poids, préférablement de 0,05 à 1,5 % en poids, le plus préférablement de 0,1 à 1 % en poids, sur la base du poids total de la composition, de phytantriol, et
(ii) 0,01 à 2 % en poids, préférablement de 0,1 à 1,5 % en poids, de 0,1 à 1 % en poids, sur la base du poids total de la composition, d'une C₄₋₆alkyl-glycérine.
